# EUROPEAN PATENT SPECIFICATION

(11) **EP 2 945 597 B1**
(45) Date of publication and mention of the grant of the patent: **01.12.2021**
(21) Application number: 14701323.9
(22) Date of filing: 17.01.2014
(51) Int. Cl.: C03C 10/00, A61K 6/818, A61K 6/833, C03C 3/076, C03C 4/00

(54) **GLASS CERAMIC MATERIAL AND METHOD**
GLASKERAMIKMATERIAL UND VERFAHREN DAFÜR
MATÉRIAU EN VITROCÉRAMIQUE ET PROCÉDÉ ASSOCIÉ

(30) Priority: 18.01.2013 US 201313744626
(43) Date of publication of application: 25.11.2015
(73) Proprietor: iKNOW-WHO GmbH, 8808 Pfäffikon SZ (CH)
(72) Inventor: XIA, Wei, S-756 45 Uppsala (SE); HARRYSON, Sigvald, S-211 14 Malmö (SE); PERSSON, Cecilia, S-756 53 Uppsala (SE)
(74) Representative: AWA Sweden AB
(86) International application number: PCT/EP2014/050930
(87) International publication number: WO 2014/111535

(56) References cited:
- US-A- 3 791 808
- US-A- 5 698 482
- US-A1- 2002 197 583
- US-A1- 2007 178 220
- US-A1- 2009 221 413
- M.NOGAMI ET AL.: "ZrO2-transformation-toughened glass-ceramics prepared by the Sol-Gel process from metal alkoxides", J.AMER. CERAM.SOC., 1986, 1986, pages 99-102, XP002723319, Retrieved from the Internet: URL:http://onlinelibrary.wiley.com/doi/10. 1111/j.1151-2916.1986.tb04709.x/abstract
- MASAYUKI NOGAMI ET AL: "Hiping of ZrO2-transformation-toughened glass-ceramics prepared by the sol-gel process from metal alkoxides", JOURNAL OF MATERIALS SCIENCE LETTERS, CHAPMAN AND HALL LTD. LONDON, GB, vol. 6, no. 12, 1 December 1987 (1987-12-01), pages 1479-1480, XP001277276, ISSN: 0261-8028
- NOGAMI ET AL: "Glass preparation of the ZrO2@?SiO2 system by the sol-gel process from metal alkoxides", JOURNAL OF NON-CRYSTALLINE SOLIDS, NORTH-HOLLAND PHYSICS PUBLISHING. AMSTERDAM, NL, vol. 69, no. 2-3, 1 January 1985 (1985-01-01), pages 415-423, XP024059777, ISSN: 0022-3093, DOI: 10.1016/0022-3093(85)90043-2 [retrieved on 1985-01-01]
- TOSHIHIRO KASUGA ET AL: "PREPARATION OF ZIRCONIA-TOUGHENED BIOACTIVE GLASS-CERAMIC COMPOSITE BY SINTER-HOT ISOSTATIC PRESSING", JOURNAL OF THE AMERICAN CERAMIC SOCIETY, BLACKWELL PUBLISHING, MALDEN, MA, US, vol. 75, no. 5, 1 May 1992 (1992-05-01), pages 1103-1107, XP000292665, ISSN: 0002-7820, DOI: 10.1111/J.1151-2916.1992.TB05545.X
- NOGAMI M: "Sol-gel preparation of SiO2 glasses containing Al2O3 or ZrO2", JOURNAL OF NON-CRYSTALLINE SOLIDS, NORTH-HOLLAND PHYSICS PUBLISHING. AMSTERDAM, NL, vol. 178, 1 November 1994 (1994-11-01), pages 320-326, XP004067788, ISSN: 0022-3093, DOI: 10.1016/0022-3093(94)90301-8
- None

## Description

### TECHNICAL FIELD

The present invention relates to methods for manufacturing of a glass ceramic material. The invention further relates to products obtainable by the methods, and to glass ceramic materials obtainable by the methods. The invention further relates to glass ceramic materials and glass ceramic bodies.

### TECHNICAL BACKGROUND

Ceramic- and glass ceramic materials are used as dental materials due to their mechanical properties and aesthetics. The all-ceramic materials, such as zirconia and alumina, have the advantage of high mechanical properties, but are opaque and thus less aesthetically pleasing and more difficult to adapt to the colour of the surrounding teeth than glass ceramic materials such as lithium disilicates, which are translucent. However, the glass ceramic materials generally have a fracture toughness and flexural strength considerably lower than the all-ceramic materials do. A translucent material with improved mechanical properties is desirable for dental restoration applications. Glass ceramic materials and methods of providing them are disclosed in patents US 5698482 A, US 2009/221413 A1, US 3791808 A and US 2002/197583 and in the scientific literature: M.NOGAMI ET AL., J.AMER. CERAM.SOC., 1986, pages 99-102; MASAYUKI NOGAMI ET AL, J.OF MATERIALS SCIENCE LETTERS, 6 (12), 1987, pages 1479-1480; and NOGAMI ET AL, J.OF NON-CRYSTALLINE SOLIDS, 69 (2-3), 1985, pages 415-423.

Furthermore, it is desired to produce crack-free samples with desirable mechanical properties having large sizes suitable for large dental works such as bridges, for example samples larger than 1 cm³.

### SUMMARY OF THE INVENTION

Purposes of the present invention include providing solutions to problems identified with regard to prior art.

The present invention allow for efficient manufacturing of a glass ceramic material suitable for, for example, dental applications.

According to a first aspect of the present invention, there is provided a method for manufacturing of a glass ceramic material for dental applications, the method comprising: providing a first precursor comprising silicon(IV); providing a second precursor comprising zirconium(IV); hydrolyzing said first precursor and second precursor in solution; polymerizing of the hydrolysed first precursor and second precursor in a solvent, wherein polymers are formed; formation of colloids comprising said polymers; formation of a gel from said colloids; aging the gel; drying the gel; producing particles from the aged and dried gel, and; compacting and sintering the particles under formation of a glass ceramic material.

Dental applications may be, for example, dental restorations.

A glass ceramic material manufactured in accordance with the first aspect of the invention comprises nano-sized tetragonal ZrO₂ in a SiO₂ matrix.The glass ceramic material may efficiently be used for dental restorations and the material may be shaped into suitable shapes such as the shape of a tooth, teeth, dental bridges or full arches, for example by fabrication with CAD/CAM processes. The material may have several beneficial properties in the field of dental restoration, such as high translucency and high toughness. The properties make the material particularly suitable for large posterior dental restorations, such as bridges or full-arches, or crowns. Further, large bodies made of the material may be obtained, such that a single body efficiently may be used for manufacturing of several restorations, thus minimizing waste and tool wear. The particles may efficiently be compacted into a desired shape and transformed to the glass ceramic material by sintering.

According to one embodiment, said first precursor is silicon(IV) alkoxide or silicon(IV) halide, and said second precursor is zirconium(IV) alkoxide or zirconium(IV)halide.

According to one embodiment, said first precursor may be silicon(IV) alkoxide, and said second precursor may be zirconium(IV) alkoxide.

The silicon(IV) alkoxide may be selected from the group consisting of silicon(IV) methoxide, silicon(IV) ethoxide, silicon(IV) propoxide, and silicon(IV) butoxide, or combinations thereof. The zircon(IV) alkoxide may be selected from the group consisting of zircon(IV) methoxide, zircon(IV) ethoxide, zircon(IV) propoxide, and zircon(IV) butoxide, or combinations thereof.

According to one embodiment the first precursor is silicon(IV) ethoxide and the second precursor is zircon(IV) propoxide.

According to one embodiment, the halides may be selected from the group comprising fluoride, chloride, bromide or iodide, or combinations thereof. For example, the halide may be chloride.

According to one embodiment, the silicon(IV) alkoxide may be selected from the group consisting of tetralkyl orthosilicate and tetraethyl orthosilicate, and the zirconium(IV) alkoxide may be selected from the group consisting of tetralkyl zirconate and tetrapropyl zirconate.

According to one embodiment, the particle sizes of the particles are in the range of 150 to 10 micrometers, for example in the range of 100 to 10 micrometers.

According to one embodiment, the producing particles from the aged and dried gel, may be grinding or milling of the aged and dried gel wherein particles are formed. According to one embodiment the producing particles from the aged and dried gel may be by means of planetary milling.

The producing particles from the aged and dried gel may be made by any type of suitable procedure whereby particles are produced from the aged and dried gel, such as, for example, grinding, pulverizing, milling, or other suitable methods for producing powders or particles.

The glass ceramic material may be biocompatible, which makes it particularly suitable to use in certain dental applications.

The method may be described as comprising a sol-gel method, the sol-gel method comprising the steps from, and including, providing the first precursor to, and including, the drying the gel.

The polymerizing may results in a homogeneous system or a homogeneous polymer.

The aging and/or the drying of the gel may result in a continuous polymeric network . The continuous polymeric network may be a monolith, or a monolithic xerogel. Longer drying times, such as one or several weeks, eg. 1 to 4 weeks, may favor formation of the continuous polymeric network with a minimum number of cracks, or no cracks, while shorter drying times may result in a continuous polymeric network having cracks due to shrinkage during the drying. Since, particles are produced from the aged and dried gel, such cracks are of little or no concern related to the characteristics of the glass ceramic material obtained from compacting and sintering the particles. Thus, shorter drying times may be preferred, for example to reduce production times. For example, the drying time may be 1 or more days, for example, 1-5 days, or 2-3 days. The drying may take place at room temperature.

It is realized that even if a major part by weight of the produced particles is in the form of small discrete particles, the particles may also contain larger particles or other larger structures. Size fractions may be removed such as by sieving prior to the compacting and the sintering of particles, and, thus, a desired fraction can be selected for compacting and sintering. The formation of a glass ceramic material by the compacting and sintering of the particles has advantages including that the volume and shape of the sintered body is not limited to sizes of aged and dried bodies which can be made without cracks. Thus, the particles may be produced in a large amount, and optionally divided into smaller fractions for treatment into bodies of glass ceramic material. Further, the particles are compacted to a desired shape followed by sintering. Thus, machining may be minimized or avoided.

According to one embodiment, the sizes of the particles is in the range of 150 to 10 micrometers, for example in the range of 100 to 50 micrometers.

Such sizes of particles may result in efficient compacting and sintering. Smaller particles may require very high pressures during sintering and/or require very high temperatures during sintering, thus resulting in poor qualities of the resulting glass ceramic material.

The compacting may be performed at 30 MPa or higher pressures, such as in the range of 100 to 400 MPa or in the range of 50 to 100 MPa. Pressing agents, such as PEG or PVA or mixtures thereof, may be mixed with the powder before the pressing in order to improve the pressing. The total amount of PEG and PVA may be 1-20 percent by mass, such as 3-7%, or 5-10%.

According to one embodiment, the sintering may be spark plasma sintering or hot isostatic pressure sintering. For example, the sintering may be spark plasma sintering. The sintering takes place in the range of 900°C to 1200°C, for example 900°C to 1150°C, such as 1000°C to 1100°C. The high temperatures and pressures of, for example spark plasma sintering and/or hot isostatic pressure sintering may result in a glass ceramic material with particularly good properties including low porosity and small grain sizes, which may lend the glass ceramic material produced from the sintering excellent properties including a high translucency and high strength. High strength may be obtained as a result of the suitable crystal sizes of ZrO₂ crystals. Further the sintering temperatures, should not be too high which may result in higher levels of monoclinic ZrO₂ resulting in decreased undesirably low translucency of the glass ceramic material.

The glass ceramic material and bodies prepared from the glass ceramic material have a fracture toughness in the range of 3 to 5 MPa. For example, the translucency may be 50% or higher, such as 70 to 90%, or 60 to 75%. The glass ceramic material obtained from the sintering is characterized by having nano-sized tetragonal ZrO₂ in a SiO₂ matrix.

During sintering, the gel may be subjected to a pressure of, for example, 50 to 200 MPa, such as 60 to 150 MPa, during at least a part of the sintering, and the sintering may be performed in less than 60 minutes, such as below 20 minutes or in the range of 1 to 5 minutes.

During sintering, the particles being sintered may be, for example, heated at rates of up to 1000 K/min. The sintering may be preceded by heat treatment wherein solvent and/or organic matter is removed from the gel.

According to one embodiment, the sintering may be spark plasma sintering, or hot isostatic pressing sintering, wherein the sintering takes place in the range of 900°C to 1200°C, wherein the pressure may be in the range of 50 to 200 MPa.

The glass ceramic material obtained from sintering may be in the shape of a disc or a cube.

Other similar or suitable sintering techniques may alternatively be used if suitable, such as techniques known as field assisted sintering (FAST) or pulsed electric current sintering (PECS).

According to one embodiment, the method may further comprise forming a dental restoration body from the sintered glass ceramic material.

A dental restoration body, such as a body in the shape of a whole or part of a tooth, a crown, or a dental bridge, produced from the material according to the method of aspects of the invention, may have excellent properties such as high toughness, high translucency, and high strength.

According to one embodiment, the hydrolysis may be partial, to a major extent or complete.

According to one embodiment, the hydrolysing may take place separately, sequentially or simultaneously.

Thus, for example, the first precursor may be hydrolysed in a first vessel and the second precursor may be hydrolysed in a second vessel; or, the first precursor may first be hydrolysed after which the second precursor is mixed in and hydrolysed, or vice versa; or the first precursor and the second precursor may be hydrolysed at the same time in the same or in different vessels, mixed or non-mixed.

According to one embodiment, the first precursor and the second precursor may be mixed or contacted before hydrolysing.

According to one embodiment, the first precursor may be mixed or contacted with the second precursor prior to hydrolysing. According to one embodiment, the at least partially hydrolysed first precursor may be mixed with the second precursor.

According to one embodiment, the at least partially hydrolysed second precursor may be mixed with the first precursor.

According to one embodiment, the at least partially hydrolysed first precursor may be mixed with the at least partially hydrolysed second precursor.

The hydrolysing takes place in a suitable solvent.

The colloids may be spontaneously formed. The formation of a gel and the aging may take place under removal of liquid or solvent, such as by evaporation. The formation of a gel and aging may, at least partially, take place during drying.

According to one embodiment, the hydrolysing may be performed by means of an added acid, such as a strong acid, for example HCl.

According to one embodiment, the hydrolysis may last from 30 minutes to several hours, for example, 1-5 hours, or 3-4 hours.

According to one embodiment, said drying of the gel may comprise: heating in a humid environment; followed by heating in a dry environment under removal of liquid. Thus, the gel may be initially heat treated without significant loss of liquid followed by heat treating under drying conditions with loss of liquid.

According to one embodiment, the solvent may comprise a mixture of an alcohol, for example ethanol, and water.

According to one embodiment the first precursor may be provided or hydrolysed in a solvent comprising or to a major part consisting of alcohol, preferably ethanol, such as 95% ethanol.

According to one embodiment the second precursor may be provided or hydrolysed in a solvent comprising or to a major part consisting of alcohol, preferably propanol, more preferably 1-propanol.

According to one embodiment, 25-50% of the oxides of the glass ceramic material are derived from the second precursor.

According to one embodiment, the molar ratio between the second precursor and the first precursor is in the range of 20/80 to 60/40, preferably 30/70 to 40/60, for example about 35/65 Thus, the molar ratio between zirconium(IV) and silicon(IV) is in the range of 20/80 to 60/40, preferably 30/70 to 40/60, such as for example about 35/65.

According to one embodiment the method may comprise: providing silicon(IV) alkoxide; providing zirconium(IV) alkoxide or zirconium(IV) chloride; hydrolyzing the silicon(IV) alkoxide, and zirconium(IV) alkoxide or zirconium(IV) chloride, in solution by means of acid; polymerizing of the hydrolysed silicon(IV) alkoxide, and zirconium(IV) alkoxide or zirconium(IV) chloride in a solvent by polycondensation, wherein polymers are formed; formation of colloids comprising said polymers; formation of a gel from said colloids; aging the gel; drying the gel, grinding of the aged and dried gel such that particles are formed; and compacting and sintering the particles from the grinding by means of spark plasma sintering or hot isostatic sintering under formation of a glass ceramic material.

According to one embodiment, the silicon alkoxide may be tetraethyl orthosilicate and the zirconium(IV) alkoxide or zirconium(IV) chloride may be zirconium propoxide; the hydrolyzing of the tetraethyl orthosilicate may be performed by addition of HCI-solution followed by stirring and provision of the zirconium propoxide followed by addition of HCI. The aging the gel and drying the gel may be performed at room temperature for 2-5 days and at 100°C to 200°C for 2 to 5 days. The aged and dried gel may be subjected to grinding by means of a planetary milling machine followed by sieving such that particles with particle sizes below 100 micrometers are obtained, the obtained particles may be compacted and sintered by spark plasma sintering The spark plasma sintering may be performed at a pressure in the range of 40 to 60 MPa, for example, 60 MPa, at temperatures in the range of 1000°C to 1200°C, for example about 1150°C and a holding time of about 5 minutes, and a ramping rate of about 120°C per minute up to about 900°C and about 30°C per minute above about 900°C.

According to a second aspect, there is provided a method for manufacturing of a glass ceramic material for dental applications, the method comprises: providing an aged and dried gel comprising silicon(IV) and zirconium(IV); producing particles from the aged and dried gel; and compacting and sintering the particles under formation of a glass ceramic material. The aged and dried gel may be a gel obtained from or obtainable from a sol-gel process. For example, the aged and dried gel may be obtained according to parts of a method according to the first aspect of the invention.

According to a third aspect, there is provided a glass ceramic material for dental applications obtainable by the method according to the first or second aspects. According to one embodiment, the glass ceramic material may be used in dental restorations. According to one embodiment, the glass ceramic material may be used in dental restorations, for example, dental restorations may be manufactured from the glass ceramic material. The glass ceramic material with its properties, for example related to strength and translucency, will lend the product excellent dental properties.

According to a fourth aspect, there is provided a glass ceramic material for dental applications comprising ZrO₂-SiO₂, wherein the molar ratio between ZrO₂ and SiO₂ is in the range of 30/70 to 40/60, and the flexural strength is in the range of 400 to 550 MPa.

For example the flexural strength may be about 400 MPa or higher, such as for example in the range of 400 to 600 MPa, 400 to 550 MPa, or 500 MPa or higher, such as for example about 509 MPa. The fracture toughness of the material is in the range of 3 to 5 MPa, such as in the range of 3.5 to 5 MPa.

The molar ratios between ZrO₂ and SiO₂ provides excellent bending strength and fracture toughness, if the level of ZrO₂ is too low the strengths will be too low and if the level is too high the obtained samples will have lower translucencies.

The glass ceramic material is further characterised by that the material comprises grains having an average size in the range of 10 to 100 nm, more preferably 20 to 40 nm. The grains essentially consist of crystals containing zirconia, and the grains are comprised in a silicon comprising matrix. In the invention, the glass ceramic material comprises nano-sized tetragonal ZrO₂ crystals in a SiO₂ matrix, wherein the sizes of the ZrO₂ crystals are in the range of 10 to 100 nm, such as 20 to 40 nm.

Such sizes give excellent properties to the glass ceramic material such as high translucency and high strength.

The glass ceramic material may further be described by having a translucency of 70% or higher, such as, for example, 70-90% or 70-85%.

Such a translucency may be particularly beneficial for dental restorations, for example as it enables the material to be efficiently dyed to a desirable colour of a tooth.

According to one embodiment of the invention, the molar ratio between ZrO₂ and SiO₂ is in the range of 30/70 to 40/60, the flexural strength of the material may be in the range of 400 to 550 MPa, such as in the range of 490 to 525, the fracture toughness of the material is in the range of 3 to 5 MPa, and the glass ceramic material is characterized by comprising tetragonal ZrO₂ crystals in a SiO₂ matrix, wherein the sizes of the ZrO₂ crystals are in the range of 10 to 100 nm, such as 20 to 40 nm. Such a material is very suitable for dental applications.

According to one embodiment the glass ceramic material may comprise to a major part ZrO₂-SiO₂. For example, 75-100% or 95-100% of the glass ceramic material may comprise ZrO₂-SiO₂. It may be preferred that the glass ceramic material essentially only consists of ZrO₂-SiO₂.

According to one embodiment, the glass ceramic material comprises grains with an average size 10 to 100 nm, more preferably 20-40 nm. The grains are tetragonal ZrO₂ crystals in a SiO₂ matrix.

According to one embodiment, the material may further comprise at least one additive. For example Li, compounds comprising Li, or Li-ions. According to one embodiment, the material may further comprise 20 m% or less of Li, preferably 1-10 m% of Li.

According to one embodiment, the additive may be at least one colouring agent, or a sintering agent, or mixtures thereof.

The glass ceramic material of the fourth aspect may be manufactured in accordance with the methods of the first and the second aspects.

According to a fifth aspect, there is provided a glass ceramic body made of the glass ceramic material according to the fifth aspect or the sixth aspect, wherein the body has a volume of at least 1 cm³, for example 1-5 cm³.

According to one embodiment, the body may be in the shape of a disc, a cube, or a rectangular parallelepiped.

According to a sixth aspect, there is provided the use of the glass ceramic material according to the third, fourth, or fifth aspects for dental restorations.

Embodiments and discussions with regard to one aspect may be relevant to one or more of the other aspects. For example embodiments and discussions with regard to the first aspect may be relevant to the second to fourth aspects. References to these embodiments are hereby made, where relevant.

### BRIEF DESCRIPTION OF THE DRAWINGS

Figure 1 illustrates heat treatment and sintering according to one embodiment.
Figure 2 illustrates an XRD pattern of a glass ceramic material according to one embodiment.
Figure 3 illustrates an XRD pattern of a glass ceramic material according to one embodiment.
Figure 4 illustrates a bright field TEM image of a glass ceramic material according to an embodiment.
Figure 5 illustrates selected area diffraction pattern.

### DETAILED DESCRIPTION

The invention will now be explained in more detail, and specific preferred embodiments, and variations of these, will be shown. The explanations are intended for illustrative and explanatory purposes and are not to be seen in any way as limiting the scope of the invention. The illustrations are schematic and all details are not illustrated, and all illustrated details may not be necessary for the invention.

A specific embodiment of the invention will now be discussed, wherein a glass ceramic material is manufactured.

ZrO₂-SiO₂ glass ceramics of 30, 35 and 40 mol% ZrO₂ were produced, using a sol-gel method. A drying control additive was used in the present study for reducing the number of cracks in the specimens. Sol-gels were produced using the alkoxide precursors tetraethyl orthosilicate (TEOS) and 70 wt.% tetrapropyl zirconate (TPZ) in 1-propanol (all chemicals were acquired from Sigma-Aldrich, St Louis, MO, USA). Synthesis was initiated by mixing ethanol (EtOH, >95%) and aqueous hydrochloric acid (HCl), for the hydrolysis, in a 50 ml round bottom flask, followed by the addition of TEOS under continuous stirring. The resulting molar ratio of the solution was 1:1:1:1 - TEOS:DMF:EtOH:H2O. The, thus, partially hydrolysed TEOS was magnetically stirred for 3 hours in order to obtain a clear sol and ensure its homogeneity. The desired amount of TPZ was then added slowly using a micropipette and magnetic stirring of the solution was continued overnight. As EtOH is volatile, the sol was kept covered during the stirring in order to minimize any changes in concentration due to evaporation. Depending on the amount of zirconium alkoxide precursor added to the solution, aqueous (0.15 M, 0.40 M or 12.18 M) HCI was then added drop by drop to initiate the final hydrolysis and polymerization of a gel. After the final synthesis step, the solution was divided and transferred to Teflon^{®} moulds of 25 mm diameter, which were sealed with polymer film for controlled evaporation. The sols were left to gel and age until approximately 40% shrinkage was observed and a stiff gel was formed. In this example,the polymer film was perforated after one week to increase the evaporation which resulted in samples that were easily detached from the mould. The samples were left to age for approximately three days at room temperature.

After formation of the gel, samples were moved to an oven and kept overnight at 110_{°}C. The temperature may be for example 90-120_{°}C; and the time may be for example 1-10 hours, or 3-6 hours. After synthesis and drying, the obtained material was wet-milled in aqueous solution during 24 hours whereby a fine powder in suspension was obtained. The suspension was filtered and the thus obtained powder was dried to eliminate water. To break up any resulting agglomerates, the material was ground and sieved (50 µm mesh). The powder was then treated by pressing into a tablet in a mould. For each tablet, approximately 0.3 g of powder was prepared into a green piece which suitable dimensions or a thickness of 1 mm and a diameter of 14 mm. Pressing aid Polyvinyl acetate was added. The powder and the pressing aid, 5% by weight, were mixed and pressed under 50 MPa, 7.7 kN, or 10 MPa, 1.5 kN, during 30 seconds.

After the pressing, Cold Isostatic Pressing (CIP) were performed on some samples to increase the green density.

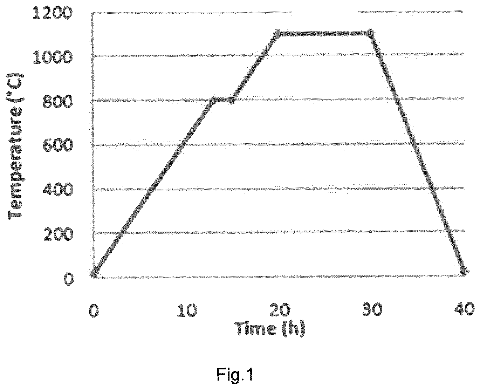

Since the drying was done during the material synthesis, only the sintering is included in the post-preparation heat treatment. A heat program with two levels were used as illustrated in figure 1: the calcination, at 800°C during 1 hour, and the sintering, at 1100°C during 10 hours. Heating rate between these stages was 60°C/hour. The calcination removes solvents and/or organic components which could be present in material and avoids cracks in sample.

The obtained glass ceramic matrial had excellent properties. According to another embodiment, the sintering may be performed using the hot isostatic pressing sintering or spark plasma sintering.

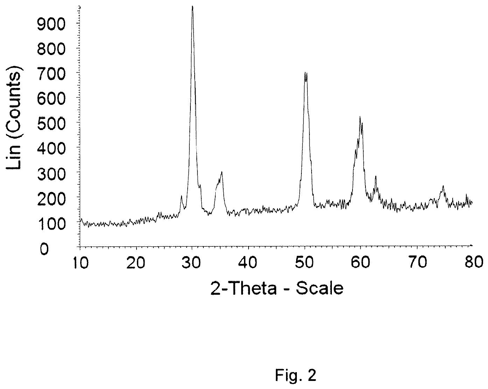

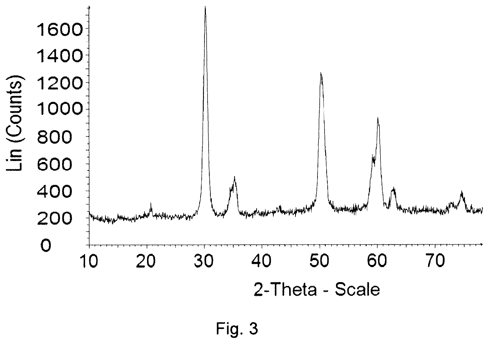

A specific embodiment of the invention will now be discussed with references to figures 2 and 3. According to this example, the particles were obtained as described with regard to the specific embodiment described above. The sintering for this embodiment was spark plasma sintering. Figures 2 and 3, illustrates results from X-ray diffraction analysis of the samples. Figure 3 illustrates the results of a sample described by having a composition of 50% ZrO₂-50%SiO₂. The peaks are tetragonal ZrO₂. Figure 3 illustrates the results of a sample described by having a composition of 35% ZrO₂-65%SiO₂. The peaks are tetragonal ZrO₂.

Another specific embodiment will now be described including preparation of and sintering of particles into a glass ceramic material. Particles were prepared through adding silicon precursor (TEOS) into ethanol (≥95%) with a molar ratio of TEOS/etOH=1:1 to 1:2, with magnetic stirring and continuously adding of 0.4 mol/l HCI solution with stirring for 0.5-3h until a clear sol was obtained. The molar ratio of HCl /etOH may be, for example, 1:1. When the thus prepared sol appears clear and thoroughly mixed, zirconium precursor (zirconium propoxide) was carefully added. The mixture was covered to avoid evaporation and magnetic stirring continued for 2-12 hours. Depending on the amount of Zr in the sol, concentrated HCI (37wt%) was added drop by drop. The volume ratio of the mixture/HCI may be, for example, 1:0.06 to 1:0.2. When all HCI has been added, the sol was transferred to a container for gelation. The gelation time may be, for example, 2 to 5 days.
The, thus, aged gel was dried at 100 to 200 °C for 2 to 5 days to make sure the such prepared gel is totally dried.

The, thus, prepared gel was then sintered according to the following: The dried gel is milled, or ground, by planetary milling machine for 1 to 2 hours. The milled powders are sieved to get a particle size below 100 micrometer, for this example in the range of 50 to 100 µm. The SPS sintering parameter were: (I) pressure: 60MPa, (II) temperature: 1150°C, the temperature may be 1000 to 1200°C, (III) holding time: 5 min, (IV) ramping rate: 120 degree/min before 900°C, and 30 degree after 900°C. The, thus, sintered samples were then polished.

If the sintering temperature is too low, the strength of the obtained material may be too low because of limits of crystallinity and crystal size of ZrO₂. If the temperature is too high, more monoclinic ZrO₂ will be formed, which may decrease the fracture toughness and the translucency. The sintering temperatures according to the embodiments, for example temperatures about 1150°C or below 1150°C, may avoid or minimise undesired phase transformation of ZrO₂ from tetragonal to monoclinic, thus resulting in high toughness of the glass ceramic material.

The molar ratio of ZrO₂:SiO₂ may be, for example, 30:70 to 40:60, such as for example 35% ZrO₂.

If the amount of ZrO₂ is too low, the mechanical strength, bending strength and fracture toughness, will be low. If the amount of ZrO₂ is too high, the obtained samples will not be translucent.

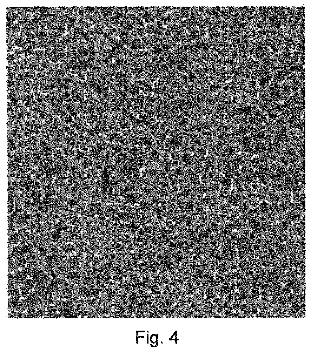

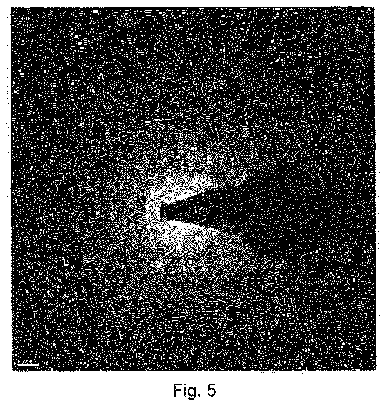

Analysis of the, thus, sintered materials were performed using a Titan 80-300 operated at 300kV recording bright-field TEM images, which show compositional contrast, and diffraction patterns, and results are illustrated in figures 4 and 5. Revealed that the material appeared very homogeneous, with small grain size on the order of less than 50 nm (about in the range of 20 to 40nm). The glass-ceramic is polycrystalline and uniform in composition.

Further it is concluded that the ZrO₂-SiO₂ glass ceramic material has tetragonal ZrO₂, small amount of Quartz, and amorphous SiO₂. The crystallized ZrO₂ not only increase the flexural strength, but also give high fracture toughness to the material. Also the phase composition, influences the mechanical strength. Crystallinity and crystal size of ZrO₂ may be key factors which influences a high strength.

Planetary milling may be efficient, for example since it may reduce manufacturing time, for example it may require only one hour, and since it may deliver a particle size within a controllable range. Now considering compacting of powders, suitable particle sizes may be between 10 and 100 µm. Particles below 10 µm may require more energy to be pressed because of the scaling of the high-friction surface area. Ball milling normally requires days to and the particle size may typically be within a wider range. Grinding may be performed until all grains are no larger than 100 µm, but grains smaller than, for example, 50 µm or 10 µm, may be avoided, eg by sieving and fractioning of the particles.

Glass ceramic material with a molar ratio of ZrO₂:SiO₂ of 35/65 were manufactured in accordance with embodiments herein described wherein a flexural strength of 509 MPa was measured on a sample measuring 15.5 mm in diameter and 2.3 mm in thickness. Such strength is very high and excellent for dental purposes.

Further, fracture toughness of the glass ceramic material according to the embodiments are high. This may for example be the result of the tetragonal phase of the zirconia crystals resulting in for example a dense structure.

## Claims

1. A method for manufacturing of a glass ceramic material comprising tetragonal ZrO₂ crystals in a SiO₂ matrix, wherein the sizes of the ZrO₂ crystals are in the range of 10 to 100 nm for dental applications, the method comprising:
- providing a first precursor comprising silicon(IV), wherein said silicon(IV) is silicon(IV) alkoxide or silicon(IV) halide,
- providing a second precursor comprising zirconium(IV), wherein said zirconium(IV) is zirconium(IV) alkoxide or zirconium(IV) halide,
wherein the molar ratio between zirconium(IV) and silicon(IV) is in the range of 20/80 to 60/40,
- hydrolyzing said first precursor and second precursor in solution,
- polymerizing of the hydrolysed first precursorand second precursor in a solvent, wherein polymers are formed,
- formation of colloids comprising said polymers
- formation of a gel from said colloids
- aging the gel,
- drying the gel,
- producing particles from the aged and dried gel, wherein the particle sizes of the particles are in the range of 150 to 10 micrometers and
- compacting and sintering the particles under formation of a glass ceramic material, wherein the sintering takes place in the range of 900°C to 1200°C, and wherein the glass ceramic material is **characterized by** comprising tetragonal ZrO₂ crystals in a SiO₂ matrix, wherein the sizes of the ZrO₂ crystals are in the range of 10 to 100 nm.

2. The method according to claim 1, wherein
the first precursor is silicon(IV) alkoxide selected from the group consisting of silicon(IV) methoxide, silicon(IV) ethoxide, silicon(IV) propoxide, and silicon(IV) butoxide, or combinations thereof, and
the second precursor is zirconium(IV) alkoxide selected from the group consisting of zircon(IV) methoxide, zircon(IV) ethoxide, zircon(IV) propoxide, and zircon(IV) butoxide, or combinations thereof.

3. The method according to claim 1 or 2, wherein the producing particles from the aged and dried gel, is grinding or milling of the aged and dried gel wherein particles are formed.

4. The method according to anyone of the previous claims, wherein the producing particles from the aged and dried gel is by means of planetary milling.

5. The method according to anyone of the previous claims, further comprising forming a dental restoration body from the sintered glass ceramic material.

6. The method according to anyone of the previous claims, wherein the hydrolysing takes place separately, sequentially or simultaneously.

7. The method according to anyone of the previous claims, wherein said drying of the gel, comprises:
heating in a humid environment, followed by
heating in a dry environment under removal of liquid.

8. The method according to anyone of the previous claims, wherein the glass ceramic material has a translucency of 70% or above.

9. The method according to anyone of the previous claims, wherein the molar ratio between zirconium(IV) and silicon(IV) is in the range of 30/70 to 40/60.

10. The method according to anyone of the previous claims, wherein said sintering is spark plasma sintering, or hot isostatic pressing sintering.

11. The method according to claim 10, wherein the pressure is in the range of 50 to 200 MPa.

12. A glass ceramic material for dental applications comprising ZrO₂-SiO₂, wherein
the molar ratio between ZrO₂ and SiO₂ is in the range of 30/70 to 40/60, and
the flexural strength of the material is in the range of 400 to 550 MPa, and the fracture toughness is in the range of 3 to 5 MPa, and
wherein the glass ceramic material is **characterized by** comprising tetragonal ZrO₂ crystals in a SiO₂ matrix, wherein the sizes of the ZrO₂ crystals are in the range of 10 to 100 nm.

## Patentansprüche

1. Verfahren für die Herstellung eines Glaskeramikmaterials umfassend tetragonale ZrO₂-Kristalle in einer SiO₂-Matrix, wobei die Größen der ZrO₂-Kristalle für zahnmedizinische Anwendungen im Bereich von 10 bis 100 nm liegen, wobei das Verfahren Folgendes umfasst:
- Bereitstellen eines ersten Vorläufers umfassend Silicium(IV), wobei das Silicium(IV) Silicium(IV)alkoxid oder Silicium(IV)halogenid ist,
- Bereitstellen eines zweiten Vorläufers umfassend Zirkonium(IV), wobei das Zirkonium(IV) Zirkonium(IV)alkoxid oder Zirkonium(IV)halogenid ist,
wobei das Molverhältnis zwischen Zirkonium(IV) und Silicium(IV) im Bereich von 20/80 bis 60/40 liegt,
- Hydrolysieren des ersten Vorläufers und zweiten Vorläufers in Lösung,
- Polymerisieren des hydrolysierten ersten Vorläufers und zweiten Vorläufers in einem Lösungsmittel, wobei Polymere gebildet werden,
- Bildung von Kolloiden umfassend die Polymere,
- Bildung eines Gels aus den Kolloiden
- Altern des Gels
- Trocknen des Gels,
- Herstellen von Partikeln aus dem gealterten und getrockneten Gel, wobei die Partikelgrößen der Partikel im Bereich von 150 bis 10 Mikrometern liegen und
- Verdichten und Sintern der Partikel unter Bildung eines Glaskeramikmaterials, wobei das Sintern im Bereich von 900 °C bis 1200 °C erfolgt und wobei das Glaskeramikmaterial **dadurch gekennzeichnet ist, dass** es tetragonale ZrO₂-Kristalle in einer SiO₂-Matrix umfasst, wobei die Größen der ZrO₂-Kristalle im Bereich von 10 bis 100 nm liegen.

2. Verfahren nach Anspruch 1, wobei
der erste Vorläufer Silicium(IV)alkoxid ist ausgewählt aus der Gruppe bestehend aus Silicium(IV)methoxid, Silicium(IV)ethoxid, Silicium(IV)propoxid und Silicium(IV)butoxid oder Kombinationen davon und
der zweite Vorläufer Zirkonium(IV)alkoxid ist ausgewählt aus der Gruppe bestehend aus Zirkon(IV)methoxid, Zirkon(IV)ethoxid, Zirkon(IV)propoxid und Zirkon(IV)butoxid oder Kombinationen davon.

3. Verfahren nach Anspruch 1 oder 2, wobei das Herstellen von Partikeln aus dem gealterten und getrockneten Gel Mahlen oder Vermahlen des gealterten und getrockneten Gels ist, wobei Partikel gebildet werden.

4. Verfahren nach einem der vorhergehenden Ansprüche, wobei das Herstellen von Partikeln aus dem gealterten und getrockneten Gel durch planetäres Mahlen erfolgt.

5. Verfahren nach einem der vorhergehenden Ansprüche, ferner das Bilden eines Zahnrestaurationskörpers aus dem gesinterten Glaskeramikmaterial umfassend.

6. Verfahren nach einem der vorhergehenden Ansprüche, wobei das Hydrolysieren einzeln, sequenziell oder gleichzeitig erfolgt.

7. Verfahren nach einem der vorhergehenden Ansprüche, wobei das Trocknen des Gels Folgendes umfasst:
Erhitzen, in einer feuchten Umgebung, gefolgt von
Erhitzen in einer trockenen Umgebung unter Entfernung von Flüssigkeit.

8. Verfahren nach einem der vorhergehenden Ansprüche, wobei das Glaskeramikmaterial eine Lichtdurchlässigkeit von 70 % oder darüber aufweist.

9. Verfahren nach einem der vorhergehenden Ansprüche, wobei das Molverhältnis zwischen Zirkonium(IV) und Silicium(IV) im Bereich von 30/70 bis 40/60 liegt.

10. Verfahren nach einem der vorhergehenden Ansprüche, wobei das Sintern Funkenplasmasintern oder isostatisches Heißpresssintern ist.

11. Verfahren nach Anspruch 10, wobei der Druck im Bereich von 50 bis 200 MPa liegt.

12. Glaskeramikmaterial für zahnmedizinische Anwendungen umfassend ZrO₂-SiO₂, wobei
das Molverhältnis zwischen ZrO₂ und SiO₂ im Bereich von 30/70 bis 40/60 liegt und
die Biegefestigkeit des Materials im Bereich von 400 bis 550 MPa liegt und die Bruchzähigkeit im Bereich von 3 bis 5 MPa liegt und
wobei das Glaskeramikmaterial **dadurch gekennzeichnet ist, dass** es tetragonale ZrO₂-Kristalle in einer SiO₂-Matrix umfasst, wobei die Größen der ZrO₂-Kristalle im Bereich von 10 bis 100 nm liegen.

## Revendications

1. Procédé de fabrication d'un matériau vitrocéramique comprenant des cristaux tétragonaux de ZrO₂ dans une matrice de SiO₂, dans lequel les tailles des cristaux de ZrO₂ sont comprises dans la plage allant de 10 à 100 nm pour des applications dentaires, le procédé comprenant :
- la fourniture d'un premier précurseur comprenant du silicium (IV), dans laquelle ledit silicium (IV) st un alcoxyde de silicium (IV) ou un halogénure de silicium (IV),
- la fourniture d'un second précurseur comprenant du zirconium (IV), dans laquelle ledit zirconium (IV) est un alcoxyde de zirconium (IV) ou un halogénure de zirconium (IV),
dans lequel le rapport molaire entre le zirconium (IV) et le silicium (IV) est compris dans la plage allant de 20/80 à 60/40,
- l'hydrolyse desdits premier précurseur et second précurseur en solution,
- la polymérisation des premier précurseur et second précurseur hydrolysés dans un solvant, dans laquelle les polymères sont formés,
- la formation de colloïdes comprenant lesdits polymères
- la formation d'un gel à partir desdits colloïdes
- le vieillissement du gel,
- le séchage du gel,
- la production de particules à partir du gel vieilli et séché, dans laquelle les granulométries des particules sont comprises dans la plage allant de 150 à 10 micromètres et
- le compactage et le frittage des particules pour former un matériau vitrocéramique, dans lesquels le frittage ayant lieu dans la plage de 900 °C à 1200 °C, et dans lequel le matériau vitrocéramique est **caractérisé en ce qu'**il comprend des cristaux tétragonaux de ZrO₂ dans une matrice de SiO₂, dans lequel les tailles des cristaux de ZrO₂ sont comprises dans la plage allant de 10 à 100 nm.

2. Procédé selon la revendication 1, dans lequel
le premier précurseur est un alcoxyde de silicium (IV) sélectionné dans le groupe constitué par le méthoxyde de silicium (IV), l'éthoxyde de silicium (IV), le propoxyde de silicium (IV) et le butoxyde de silicium (IV) , ou des combinaisons de ceux-ci, et
et le second précurseur est un alcoxyde de zirconium (IV) sélectionné dans le groupe constitué par le méthoxyde de zirconium (IV), l'éthoxyde de zirconium (IV), le propoxyde de zirconium (IV) et le butoxyde de zirconium (IV) , ou des combinaisons de ceux-ci.

3. Procédé selon la revendication 1 ou 2, dans lequel la production de particules à partir du gel vieilli et séché est le broyage ou le fraisage du gel vieilli et séché dans lequel des particules sont formées.

4. Procédé selon l'une quelconque des revendications précédentes, dans lequel la production de particules à partir du gel vieilli et séché se fait au moyen d'un broyage planétaire.

5. Procédé selon l'une quelconque des revendications précédentes, comprenant en outre la formation d'un corps de restauration dentaire à partir du matériau vitrocéramique fritté.

6. Procédé selon l'une quelconque des revendications précédentes, dans lequel l'hydrolyse a lieu séparément, séquentiellement ou simultanément.

7. Procédé selon l'une quelconque des revendications précédentes, dans lequel ledit séchage du gel comprend :
le chauffage dans un environnement humide, suivi par
le chauffage dans un environnement sec pour éliminer le liquide.

8. Procédé selon l'une quelconque des revendications précédentes, dans lequel le matériau vitrocéramique a une translucidité de 70 % ou plus.

9. Procédé selon l'une quelconque des revendications précédentes, dans lequel le rapport molaire entre le zirconium (IV) et le silicium (IV) est compris dans la plage allant de 30/70 à 40/60.

10. Procédé selon l'une quelconque des revendications précédentes, dans lequel ledit frittage est un frittage par plasma à étincelles ou un frittage par pression isostatique à chaud.

11. Procédé selon la revendication 10, dans lequel la pression est comprise dans la plage allant de 50 à 200 MPa.

12. Matériau vitrocéramique pour des applications dentaires comprenant du ZrO₂-SiO₂, dans lequel
le rapport molaire entre ZrO₂ et SiO₂ est compris dans la plage allant de 30/70 à 40/60, et
la résistance à la flexion du matériau est comprise dans la plage allant de 400 à 550 MPa, et la ténacité à la rupture est comprise dans la plage allant de 3 à 5 MPa, et
dans lequel le matériau vitrocéramique est **caractérisé en ce qu'**il comprend des cristaux tétragonaux de ZrO₂ dans une matrice de SiO₂, dans lequel les tailles des cristaux de ZrO₂ sont comprises dans la plage allant de 10 à 100 nm.
